# EUROPEAN PATENT SPECIFICATION

(11) **EP 4 426 248 B1**
(45) Date of publication and mention of the grant of the patent: **20.08.2025**
(21) Application number: 22803354.4
(22) Date of filing: 04.11.2022
(51) Int. Cl.: A61F 5/44

(54) **A WASTE COLLECTION BAG**
EINEN ABFALLSAMMELSACK
UN SAC DE COLLECTE DES DÉCHETS

(30) Priority: 05.11.2021 GB 202115911
(43) Date of publication of application: 11.09.2024
(73) Proprietor: Salts Healthcare Limited, West Midlands B7 4AA (GB)
(72) Inventor: JASICKA, Ewa, Birmingham, West Midlands B7 4AA (GB)
(74) Representative: Forresters IP LLP
(86) International application number: PCT/GB2022/052794
(87) International publication number: WO 2023/079303

(56) References cited:
- WO-A1-2019/083104
- WO-A1-2020/125908
- WO-A1-2021/165703
- WO-A2-2006/052587
- GB-A- 2 322 079
- US-A- 4 100 953
- US-A- 4 743 236
- US-A1- 2011 085 746

## Description

### Introduction

Embodiments of the invention relate to a waste collection bag.

Ostomy appliances are well known medicals devices for users that have a stoma. Generally, the appliance has an opening for the stoma to be received in and an adhesive member that contacts the body and holds the appliance in position. Waste exiting the stoma is collected in a collecting volume which is formed from two flexible walls connected together. Often the appliance includes an outlet valve, so that a user can empty waste that is collected in the collecting volume without having to remove and dispose of the entire appliance.

At times, a user may want to extend the time between emptying their appliance, for example, when they are sleeping. Typically, a larger capacity bag is used to contain waste while the user is sleeping. In this scenario, the outlet valve is connected to the larger capacity bag by a tube, so that the bag fills with waste.

US4743236 relates to a urine collection device that can be hung selectively from either of the two hangers located on the edges of the device. WO2020/125908 relates to a urinary catheter assembly comprising a urinary collecting bag and catheter. US2011/085746 relates to a feed bag.

WO2006/052587 relates to a free-standing urine collection bag and discloses the preamble of claim 1.

Embodiments of the present invention seek to alleviate one or more problems associated with the prior art.

According to an aspect of the invention we provide a waste collection bag for connection to an ostomy appliance including:
a first wall and a second wall, which are connected to form a pouch arrangement including a collecting volume,
an inlet, for connection to the ostomy appliance, and which inlet permits waste into collecting volume, and
a support system, wherein the collecting volume is formed in a lower portion of the pouch arrangement and the support system is formed in an upper portion of the pouch arrangement, the support system having:
   a first attachment zone including an opening,
   a second attachment zone including an opening, and
   a flexible portion which extends across the pouch arrangement and is formed where the first and second walls are not connected to together, and which flexible portion extends between the first and second attachment zones so that the first attachment zone is closer to the collecting volume than the second attachment zone.

According to a second aspect of the invention we also provide a waste collection system including a stand or holder and a waste collection bag in accordance with the first aspect.

Further optional features of the invention are provided in the appended claims.

In order that the present disclosure may be more readily understood, preferable embodiments thereof will now be described, by way of example only, with reference to the accompanying drawings, in which:
FIGURE 1 is a waste collection bag in accordance with embodiment of the present disclosure;
FIGURE 2 is the waste collection bag supported on a stand; and
FIGURES 3 and 4 are illustrations of other stand designs that the waste collection is compatible with.

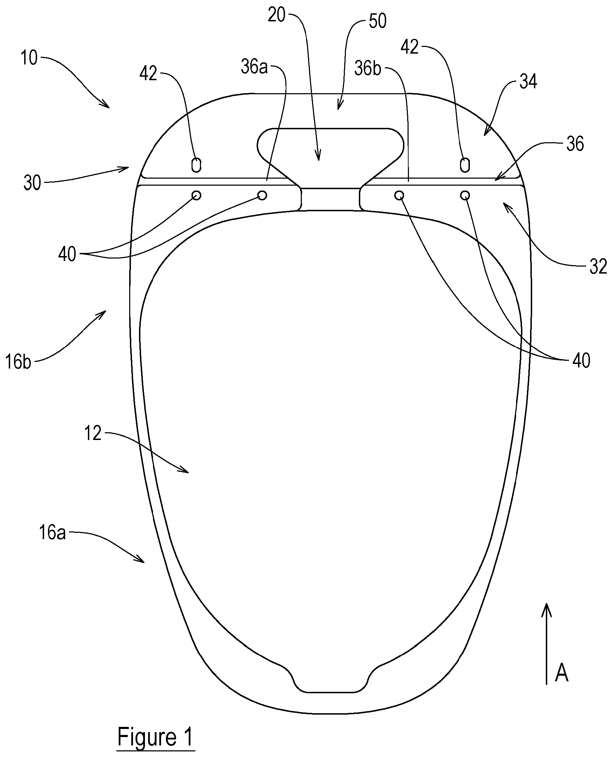

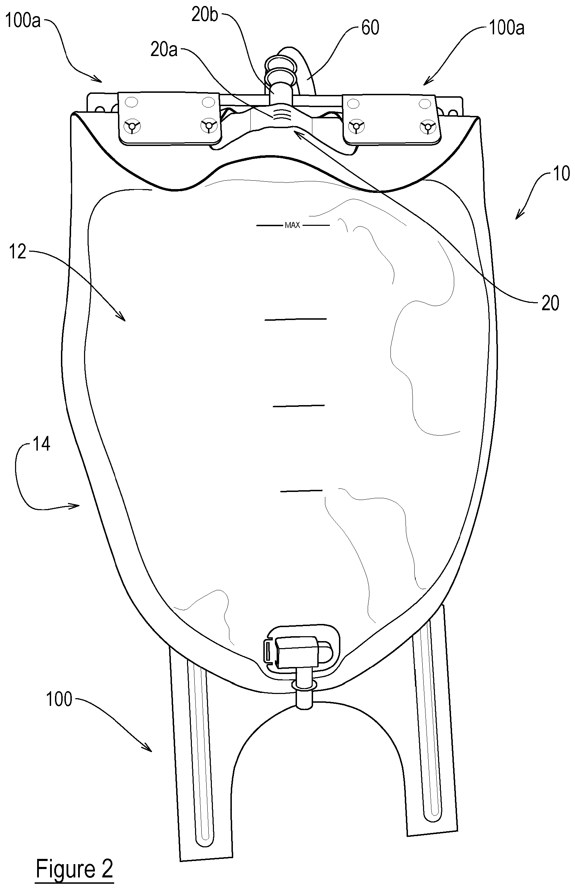

Referring to figures 1 and 2, a waste collection bag 10 for connection to an ostomy appliance (not shown) is illustrated. The waste collection bag 10 includes a first wall 12, a second wall 14, an inlet 20 and a support system 30.

The first wall 12 and the second wall 14 are connected to form a pouch arrangement which includes a collecting volume. In other words, the first and second wall 12, 14 and the connection between them defines and internal volume in which waste is collected. In the present example, the collecting volume is around two litres but it should be appreciated that the bag 10 can be made to form the desired volume within.

The collecting volume is formed in a lower portion 16a of the pouch arrangement and the support system 30 is formed in an upper portion 16b of the pouch arrangement. In the present example, the collection bag 10 is between about 37 and 38cm long (and preferably around 37.6cm long in total and the collecting volume extends from a base of the collection bag 10 upwards, to between about 75% and 85% of the total length. The remaining area at the top of the collection bag 10 (the upper portion) includes the support system 30 that is used to support the collection bag 10 on an appropriate stand, holder or hanger.

The inlet 20 is fluidly connected to the collecting volume, so that waste can flow from a connected device or appliance and into the collecting volume. In use, the inlet 20 is connected to an ostomy appliance (usually a urostomy appliance) via a tube / conduit, so that waste can flow out of a user's appliance (that is adhered about their stoma) and through the connecting tube and into the waste collection bag 10.

In some embodiments, the inlet 20 includes a body having two parts. The first part 20a is attached between the first and second walls 12, 14. The second part 20b extends away from the first part 20a and is configured to connect to a tube / conduit 60.

As can be seen from the illustrated example in figure 2, the inlet 20 is provided in a generally central region of the upper portion 16b of the pouch arrangement. In use, the connected tube / conduit 60 extends away from the collecting volume.

The support system 30 includes a first attachment zone 32, a second attachment zone 34, and a flexible portion 36. The first attachment zone 32 includes an opening 40 and the second attachment zone 34 includes an opening 42. Further, the first attachment zone 32 is closer to the collecting volume than the second attachment zone 34. The flexible portion 36 extends between the first and second attachment zones 32, 34. The term flexible portion is used to describe a region that is relatively more flexible than the area or regions around it.

In some embodiments, the first attachment zone 32 includes multiple openings 40. Referring to figure 1, the first attachment zone 32 includes four openings 40. The openings 40 are spaced across the upper portion of the pouch arrangement. In the illustrated example, the upper portion 16b of the pouch arrangement is between around 24 and 25cm wide (and preferably between around 24.6cm). The "outer" openings 40 at either side of the first attachment zone 32 (i.e. those closest to the edge of the collection bag 10) are around 4cm from the edge. The "inner" openings 40 are around 4cm from the outer openings 40. The distance between the "inner" openings 40 is around 8cm.

In some embodiments, one or more of the openings 40 of the first attachment zone 32 are generally circular (and, in this example, are around 4.5mm in diameter).

In some embodiments, the second attachment zone 34 includes multiple openings 42. Referring to the example in figure 1, the second attachment zone 34 includes two openings 42. The openings 42 are each around 4cm from the edge of the collection bag 10 and about 16cm apart. In some embodiments, the openings 42 are slots (i.e. elongate apertures) that are around 6mm long (i.e. in a lengthwise direction A of the collection bag 10) and 4mm wide
In some embodiments, the openings 42 of the second attachment zone 34 are substantially aligned with the outer openings 40 of the first attachment zone 32. In other words, in the direction A, the openings 42 of the second attachment zone 34 are above the openings 40 of the first attachment zone 32.

In some embodiments, a handle 50 is provided in the upper portion 16b of the pouch arrangement. In the illustrated example, the handle 50 provides an opening, which forms part of the second attachment zone 34 (the functionality of this is discussed in more detail later).

In some embodiments, the flexible portion 36 extends across the pouch arrangement. In the illustrated example, the flexible portion 36 is split in two parts 36a, 36b. The first part 36a extends from an edge of the upper portion 16b to the opening provided by the handle 50. The second part 36b extends from an opposing side of the opening provided by the handle to a second edge of the upper portion 16b. In other words, the flexible portion 36 extends in a substantially straight line across the upper portion 16b (this is advantageous for the folding functionality provided by the flexible portion 36 as described below). It should be appreciated that in other embodiments, the flexible portion may be formed as a single part extending across substantially the entire width of the upper portion 16b, from one edge to the other.

As the term flexible portion describes a region that is relatively more flexible than the area or regions around it. In some embodiments, the flexible portion 36 is formed by the absence of toughened or thickened material compared to the surrounding area. In this example, the first and second walls 12, 14 are permanently connected together across the area that does not include the collecting volume, which creates a single sheet of material. This creates an upper portion 16b that is reinforced and able to support the weight of the collection bag 10, even when full of waste. However, across the flexible portion 36, the first and second walls 12, 14 are not connected together. This creates a region of comparatively higher flexibility because the two sheets of material are able to bend independently rather than being constrained by having to move together. This is advantageous for allowing the collection bag 10 to adapt to multiple support stands and hangers.

In some embodiments, the first and second walls 12, 14 are welded (preferably heat welded) about their peripheries in the lower portion 16a of the pouch arrangement and the area of the upper portion 16a is more extensively welded in order to provide a support system 30 that is strong / robust enough to support the collection bag 10 (from a hanging position) when full of waste. The flexible portion 36 is a line that is formed across the upper portion 16b between two areas that are substantially completely welded together (i.e. either side of the line is welded across the bag 10). The line remains unwelded (as described as being advantageous above), so that the first and second wall 12, 14 can fold along the line easily. In other words, the flexible portion 36 is provided in a generally straight elongate area across the bag. The relatively less flexible areas are provided both above and below the flexible portion 36. Each of the less flexible areas extends adjacent the flexible portion 36 across the bag 10. It should be appreciated that is the bag 10 includes a handle 50, the flexible portion 36 may be interrupted in a central region for an opening that provides that handle 50. In such an example, the flexible portion 36 and the less flexible areas above and below are formed in two parts that extend either side of the handle 50.

The flexible portion 36 and the first and second attachment zones 32, 34 are all located in an upper portion of the collection bag 10. In other words, they are all located in a position above the main collecting volume (when the bag 10 is in use). It should be appreciated that the inlet 20 is generally centrally located and may be positioned just below the flexible portion 36.

The waste collection bag 10 is adapted to provide at least two different configurations for connecting to more than one type of support stand, holder or hanger.

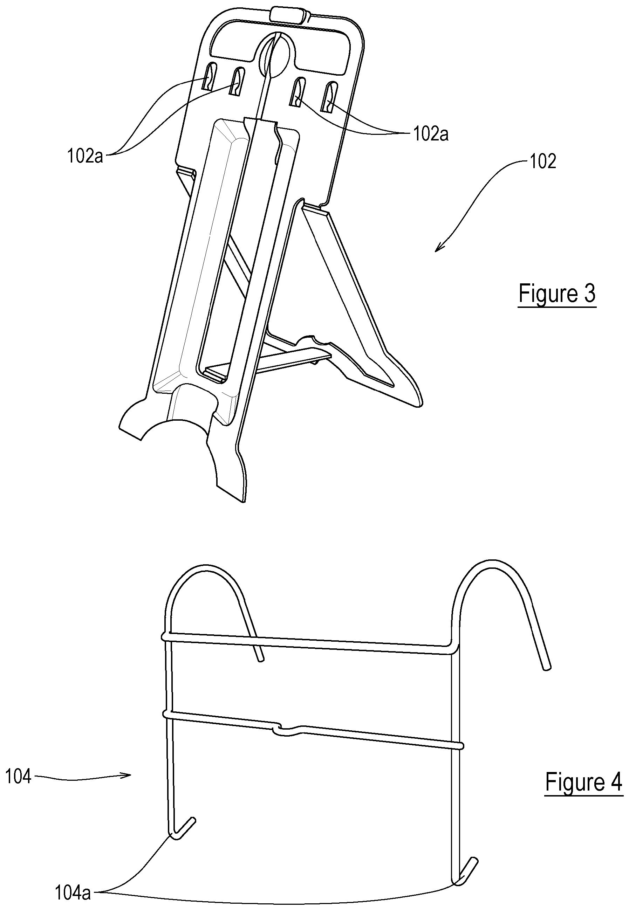

Figure 2 illustrates the collection bag 10 on a "pinch" stand 100. Figures 3 and 4 illustrate two other types of stand / hanger that the collection bag 10 is also compatible with.

The stand 100 includes four pins that extend outwards, over which the four openings 40 of the first attachment zone 32 are placed. In other words, the pins extend through the openings 40. The collection bag 10 is folded along the flexible portion 36. This brings the openings 42 of the second attachment zone 34 into contact with the pins also. Thus, the pins extend through the openings 42 of the second attachment zone 34 as well as those of the first attachment zone 32. Further, because the opening provided by the handle 50 flares outwards, this opening is positioned over the pins when the flexible portion 36 is folded. This allows a pinch piece 100a on each side of the stand 100 to be folded down and over the pins to secure the collection bag 10 in place. In other words, the first configuration of the collection bag 10 includes folding along the flexible portion 36, so that both the first attachment zone 32 and the second attachment zone 34 are used to engage the supporting stand 100.

In some embodiments, a stand includes four upward projecting hooks (see the stand 102 illustrated in figure 3). There are four hooks 102a along a back plate of the stand 102. In this example, the four openings 40 of the first attachment zone 32 are used to hang the collection bag 10. The remainder of the upper portion 16b of the pouch arrangement is supported by the back plate of the stand 102 and a connected tube 60 is arranged upwards.

Alternative stands with four upward hooks may not include the back plate. In this case, the remainder of the upper portion 16b of the pouch arrangement will bend over the top of the stand.

In some embodiments, a hanger may be used (for example, in a hospital setting where bags are more likely to be hung from the side of a bed). These types of hangers 104 (one of which is shown in figure 4), have two wide spaced hooks 104a. The outer openings 40 of the first attachment zone 32 or the openings 42 of the second attachment zone 34 could be used for hanging the collection bag 10 from a two hook hanger. It should be appreciated that if the hanger had four hooks, the opening 40 of the first attachment zone 32 would be compatible with that.

In general terms, the second of the configurations includes using only the first attachment zone 32 or the second attachment zone 34 to engage the supporting stand or hanger 102, 104.

Further, the handle 50 provides an additional way in which the collection bag 10 can be supported.

This allows a user to carry the collection bag 10 between locations. Furthermore, the handle 50 provides a larger opening that may allow the user to hang the collection bag 10 when it is not being used. For example, they may wish to hang it in a shower cubicle or another environment when the collection bag 10 has been cleaned and they are allowing it to dry out.

In some embodiments, the material of the waste collection bag 10 preferably does not include polyvinyl chloride (PVC). For example, a suitable material may be polyphenylene ether (PPE) or a thermoplastic polyurethane (TPU) material or a non-coated polyethylene (PE) may be preferred. A suitable PE may be low density polyethylene (LDPE), medium density polyethylene (MDPE), be high density polyethylene (HDPE) or a combination of two or more of them.

In should be appreciated that the first and second wall 12, 14 and the inlet 20 will preferably be made of similar and / or compatible materials. This ensures that the inlet 20 seals to the wall 12, 14 with a reliable and consistent seal when it is welded.

When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Although certain example embodiments of the invention have been described, the scope of the appended claims is not intended to be limited solely to these embodiments.

## Claims

1. A waste collection bag (10) for connection to an ostomy appliance including:
a first wall (12) and a second wall (14), which are connected to form a pouch arrangement including a collecting volume,
an inlet (20), for connection to the ostomy appliance, and which inlet (20) permits waste into collecting volume, and
a support system (30), wherein the collecting volume is formed in a lower portion (16a) of the pouch arrangement and the support system (30) is formed in an upper portion (16b) of the pouch arrangement, the support system (30) having:
a first attachment zone (32) including an opening (40),
a second attachment zone (34) including an opening (42),
**characterized by**
a flexible portion (36) which extends across the pouch arrangement and is formed where the first (12) and second walls (14) are not connected together, and which flexible portion (36) extends between the first (32) and second attachment zones (34) so that the first attachment zone (32) is closer to the collecting volume than the second attachment zone (34).

2. A waste collection bag (10) according to claim 1 wherein the first and / or the second attachment zones (34) include multiple openings (40, 42).

3. A waste collection bag (10) according to claim 2 wherein the first attachment zone (32) includes four openings (40), and / or wherein the second attachment zone (34) includes two or three openings (42).

4. A waste collection bag (10) according to any of the preceding claims wherein one or more of the openings (42) of the second attachment zone (34) is a slot.

5. A waste collection bag (10) according to any of the preceding claims wherein the upper portion (16b) includes a handle, and optionally, wherein the handle includes an opening in the upper portion (16b).

6. A waste collection bag (10) according to claim 8 wherein the handle opening forms one or more of the openings (42) of the second attachment zone (34).

7. A waste collecting bag (10) according to any of the preceding claims where dependent on any of claims 6 to 9 wherein the flexible portion (36) is split in two parts and the first part extends from an edge of the upper portion (16b) to the handle opening (40) and the second part extends from an opposing side of the handle opening to the or a second edge of the upper portion (16b).

8. A waste collection bag (10) according to any of the preceding claims wherein the upper portion (16b) is formed from the first and second wall (14) permanently attached together, and optionally, wherein the permanent attachment is formed by a heat weld.

9. A waste collection bag (10) according to any of the preceding claims wherein the inlet (20) includes a body, which includes a first part attached between the first (12) and second walls (14) a second part that extends away from the first part and is configured to connect to a tube / conduit (60).

10. A waste collection bag (10) according to claim 9 wherein the inlet (20) is provided in a generally central region of the upper portion (16b) of the pouch arrangement, so that when a tube / conduit (60) is attached, the tube / conduit (60) extends away from the collecting volume.

11. A waste collection bag according to any preceding claim including a first configuration in which the flexible portion (36) is folded so that both the first attachment zone (32) and the second attachment zone (34) are adapted to engage a supporting stand or holder (100).

12. A waste collection bag (10) according to any preceding claim including a second configuration provided only by the first attachment zone (32) or the second attachment zone (34) to engage a supporting stand or holder (100).

13. A waste collection bag (10) according to any preceding claim including a third configuration provided by a or the handle to engage a supporting stand or holder (100).

14. A waste collection system (10) including a stand or holder (100) and a waste collection bag (10) according to any of the preceding claims.

## Patentansprüche

1. Abfallstoffsammelbeutel (10) zur Verbindung mit einer Stomavorrichtung, einschließend:
eine erste Wand (12) und eine zweite Wand (14), die verbunden sind, um eine Folienbeutelanordnung zu bilden, die ein Sammelvolumen einschließt,
einen Einlass (20) zur Verbindung mit der Stomavorrichtung, und wobei dieser Einlass (20) Abfallstoffe in ein Sammelvolumen zulässt, und
ein Tragesystem (30), wobei das Sammelvolumen in einem unteren Abschnitt (16a) der Folienbeutelanordnung ausgebildet ist und das Tragesystem (30) in einem oberen Abschnitt (16b) der Folienbeutelanordnung ausgebildet ist, wobei das Tragesystem (30) aufweist:
eine erste Anbringungszone (32), einschließend eine Öffnung (40),
eine zweite Anbringungszone (34), einschließend eine Öffnung (42), **gekennzeichnet durch**
einen flexiblen Abschnitt (36), der sich über die Folienbeutelanordnung erstreckt und dort ausgebildet ist, wo die erste (12) und die zweite Wand (14) nicht miteinander verbunden sind, und wobei sich dieser flexible Abschnitt (36) zwischen der ersten (32) und der zweiten Anbringungszone (34) erstreckt, so dass die erste Anbringungszone (32) näher zu dem Sammelvolumen ist als die zweite Anbringungszone (34).

2. Abfallstoffsammelbeutel (10) nach Anspruch 1, wobei die erste und/oder die zweite Anbringungszone (34) mehrere Öffnungen (40, 42) einschließen.

3. Abfallstoffsammelbeutel (10) nach Anspruch 2, wobei die erste Anbringungszone (32) vier Öffnungen (40) einschließt und/oder wobei die zweite Anbringungszone (34) zwei oder drei Öffnungen (42) einschließt.

4. Abfallstoffsammelbeutel (10) nach einem der vorhergehenden Ansprüche, wobei eine oder mehrere der Öffnungen (42) der zweiten Anbringungszone (34) ein Schlitz sind.

5. Abfallstoffsammelbeutel (10) nach einem der vorhergehenden Ansprüche, wobei der obere Abschnitt (16b) einen Griff einschließt und optional wobei der Griff eine Öffnung in dem oberen Abschnitt (16b) einschließt.

6. Abfallstoffsammelbeutel (10) nach Anspruch 8, wobei die Grifföffnung eine oder mehrere der Öffnungen (42) der zweiten Anbringungszone (34) bildet.

7. Abfallstoffsammelbeutel (10) nach einem der vorhergehenden Ansprüche bei Abhängigkeit von einem der Ansprüche 6 bis 9, wobei der flexible Abschnitt (36) in zwei Teile aufgeteilt ist und sich der erste Teil von einem Rand des oberen Abschnitts (16b) zu der Grifföffnung (40) erstreckt und sich der zweite Teil von einer entgegengesetzten Seite der Grifföffnung zu dem oder einem zweiten Rand des oberen Abschnitts (16b) erstreckt.

8. Abfallstoffsammelbeutel (10) nach einem der vorhergehenden Ansprüche, wobei der obere Abschnitt (16b) aus der ersten und der zweiten Wand (14) gebildet ist, die dauerhaft aneinander angebracht sind, und optional wobei die dauerhafte Anbringung durch eine Wärmeschweißnaht gebildet wird.

9. Abfallstoffsammelbeutel (10) nach einem der vorhergehenden Ansprüche, wobei der Einlass (20) einen Körper einschließt, der einen ersten Teil, der zwischen der ersten (12) und der zweiten Wand (14) angebracht ist, einen zweiten Teil, der sich von dem ersten Teil weg erstreckt, einschließt und dazu konfiguriert ist, einen Schlauch/eine Leitung (60) zu verbinden.

10. Abfallstoffsammelbeutel (10) nach Anspruch 9, wobei der Einlass (20) in einer allgemein zentralen Region des oberen Abschnitts (16b) der Folienbeutelanordnung vorgesehen ist, so dass, wenn ein Schlauch/eine Leitung (60) angebracht wird, sich der Schlauch/die Leitung (60) von dem Sammelvolumen weg erstreckt.

11. Abfallstoffsammelbeutel nach einem vorhergehenden Anspruch, einschließend eine erste Konfiguration, in der der flexible Abschnitt (36) gefaltet ist, so dass sowohl die erste Anbringungszone (32) als auch die zweite Anbringungszone (34) dazu eingerichtet sind, einen Trageständer oder eine Tragehalterung (100) in Eingriff zu nehmen.

12. Abfallstoffsammelbeutel (10) nach einem vorhergehenden Anspruch, einschließend eine zweite Konfiguration, die nur durch die erste Anbringungszone (32) oder die zweite Anbringungszone (34) bereitgestellt wird, um einen Trageständer oder eine Tragehalterung (100) in Eingriff zu nehmen.

13. Abfallstoffsammelbeutel (10) nach einem vorhergehenden Anspruch, einschließend eine dritte Konfiguration, die durch einen oder den Griff bereitgestellt wird, um einen Trageständer oder eine Tragehalterung (100) in Eingriff zu nehmen.

14. Abfallstoffsammelsystem (10), einschließend einen Ständer oder eine Halterung (100) und einen Abfallstoffsammelbeutel (10) nach einem der vorhergehenden Ansprüche.

## Revendications

1. L'invention concerne un sac de collecte de déchets (10) destiné à être relié à un appareillage de stomie comprenant :
une première paroi (12) et une seconde paroi (14), reliées pour former un agencement de poche comprenant un volume de collecte,
une entrée (20) prévue pour être reliée à l'appareillage de stomie et par laquelle les déchets peuvent être collectés dans le volume de collecte, et
un système de support (30), dans lequel le volume de collecte est formé dans une partie inférieure (16a) de l'agencement de poche, le système de support (30) ayant :
une première zone de fixation (32) comprenant une ouverture (40),
une seconde zone de fixation (34) comprenant une ouverture (42),
**caractérisée en ce que**
une partie flexible (36) s'étendant à travers l'agencement de poche est formée au niveau de la partie non reliée entre la première paroi (12) et la seconde paroi (14), ladite partie flexible (36) s'étendant entre la première zone de fixation (32) et la seconde zone de fixation (34) de façon que la première zone de fixation (32) soit plus proche du volume de collecte que la seconde zone de fixation (34).

2. Sac de collecte de déchets (10) selon la revendication 1 dans lequel les première et/ou seconde zones de fixation (34) comprennent des ouvertures multiples (40, 42)

3. Sac de collecte de déchets (10) selon la revendication 2 dans lequel la première zone de fixation (32) comprend quatre ouvertures (40), et/ou dans lequel la seconde zone de fixation (34) comprend deux ou trois ouvertures (42).

4. Sac de collecte de déchets (10) selon l'une quelconque des revendications précédentes dans lequel une ou plusieurs des ouvertures (42) de la seconde zone de fixation (34) est une encoche.

5. Sac de collecte de déchets (10) selon l'une quelconque des revendications précédentes dans lequel la partie supérieure (16b) comprend une poignée, et de manière facultative, dans lequel la poignée comprend une ouverture dans la partie supérieure (16b).

6. Sac de collecte de déchets (10) selon la revendication 8 dans lequel l'ouverture de la poignée forme une ou plusieurs des ouvertures (42) de la seconde zone de fixation (34).

7. Sac de collecte de déchets (10) selon l'une quelconque des revendications précédentes et selon, le cas échéant, l'une quelconque des revendications 6 à 9, dans lequel la partie flexible (36) est divisée en deux parties et la première partie s'étend d'un bord de la partie supérieure (16b) à l'ouverture de la poignée (40) et la seconde partie s'étend d'un côté opposé de l'ouverture de la poignée au ou à un second bord de la partie supérieure (16b).

8. Sac de collecte de déchets (10) selon l'une quelconque des revendications précédentes dans lequel la partie supérieure (16b) est formée à partir de la première et de la seconde parois (14) fixées ensemble de manière permanente, et de manière facultative, dans lequel la fixation permanente est formée par une soudure à chaud.

9. Sac de collecte de déchets (10) selon l'une quelconque des revendications précédentes, dans lequel l'entrée (20) comprend un corps comprenant une première partie fixée entre les première (12) et seconde (14) parois, une seconde partie s'étendant à l'opposé de la première partie étant configurée pour être reliée à un tube/conduit (60).

10. Sac de collecte de déchets (10) selon la revendication 9 dans lequel l'entrée (20) est prévue dans une zone généralement centrale de la partie supérieure (16b) de l'agencement de poche, de sorte que lorsqu'un tube/conduit (60) est fixé, le tube/conduit (60) s'étend à l'écart du volume de collecte.

11. Sac de collecte de déchets selon l'une quelconque des revendications précédentes comprenant une première configuration dans laquelle la partie flexible (36) est pliée de façon que la première zone de fixation (32) et la seconde zone de fixation (34) soient adaptées à recevoir un support ou un dispositif de maintien (100).

12. Sac de collecte de déchets (10) selon l'une quelconque des revendications précédentes comprenant une seconde configuration prévue uniquement sur la première zone de fixation (32) ou la seconde zone de fixation (34) pour recevoir un support ou un dispositif de maintien (100).

13. Sac de collecte de déchets (10) selon l'une quelconque des revendications précédentes comprenant une troisième configuration prévue sur une ou la poignée pour recevoir un support ou un dispositif de maintien (100).

14. Système de collecte de déchets (10) comprenant un support ou un dispositif de maintien (100) et un sac de collecte de déchets (10) selon l'une quelconque des revendications précédentes.
